# EUROPÄISCHE PATENTSCHRIFT

(11) **EP 0 630 231 B1**
(45) Veröffentlichungstag und Bekanntmachung des Hinweises auf die Patenterteilung: **06.03.1996**
(21) Anmeldenummer: 93920536.5
(22) Anmeldetag: 16.03.1993
(51) Int. Cl.: A61K 9/127

(54) **LIPOSOMEN MIT NEGATIVER ÜBERSCHUSSLADUNG**
LIPOSOMES WITH EXCESS NEGATIVE CHARGE
LIPOSOMES A CHARGE NEGATIVE EXCEDENTAIRE

(30) Priorität: 17.03.1992 DE 4208527
(43) Veröffentlichungstag der Anmeldung: 28.12.1994
(73) Patentinhaber: Max-Planck-Gesellschaft zur Förderung der Wissenschaften e.V., D-37073 Göttingen (DE)
(72) Erfinder: EIBL, Hansjörg, D-3406 Bovenden-Eddigehausen (DE); KAUFMANN-KOLLE, Petra, D-3407 Gleichen-Diemarden (DE); KRANICH, Anneliese, D-7800 Freiburg (DE); UNGER, Clemens, D-3400 Göttingen (DE)
(74) Vertreter: Huber, Bernhard, Dipl.-Chem.
(86) Internationale Anmeldenummer: EP9300605
(87) Internationale Veröffentlichungsnummer: WO9318749

(56) Entgegenhaltungen:
- EP-A- 0 099 068
- EP-A- 0 102 324
- EP-A- 0 113 508
- WO-A-88/07362
- DE-A- 3 825 374
- US-A- 4 186 183
- P.BURI ET AL. 'formes pharmaceutiques nouvelles' 1985 , TECHNIQUE ET DOCUMENTATION (LAVOISIER) , PARIS (FR)
- PATENT ABSTRACTS OF JAPAN vol. 15, no. 216 (C-0837)1991
- ARZNEIM.-FORSCH./DRUG RES. Bd. 35, Nr. 3, März 1985, AULENDORF (DE) Seiten 587 - 592 A. TOKUMURA ET AL. 'effects of lysophosphatidic acids and their structural analogs on arterial blood pressure of cats'

## Beschreibung

Die Erfindung betrifft Liposomen mit negativer Überschußladung, insbesondere zur Verabreichung von Cytostatika und Cytokinen.

Liposomen sind kugelförmige Gebilde aus einer oder mehreren Lipiddoppelschichten mit wäßrigem Innenraum (Lipidvesikel). Derartige Bläschen lassen sich durch mechanische Feinstverteilung von Phospholipiden (z.B. Lecithin) in wäßrigen Medien herstellen.

Von Bangham et al., J.Mol.Biol. 13 (1965), 238-252, wurde beobachtet, daß Phospholipide in Gegenwart von Wasser Überstrukturen ausbilden. In Abhängigkeit von physikalischen Parametern wie Druck, Temperatur und Ionenkonzentration können sich Mizellen, unilamellare bzw. multilamellare Liposomen oder auch einfache Lipiddoppelschichten bilden (vgl. Liposomes: From physical structure to therapeutic application (1981), Knight, C.G. (ed.), Elsevier, North Holland Biomedical Press, Kapitel 2: H. Eibl, Phospholipid synthesis, 19-50; Kapitel 3: F. Szoka und D. Papahadjopoulos, Liposomes: Preparation and Characterization, 51-104). Kleine, unilamellare Liposomen sind kugelförmige Gebilde mit einem Durchmesser von 20 bis 200 nm (vgl. Barenholtz et al., FEBS Let. 99 (1979) 210-214). Ihr Innenvolumen besteht aus einem wäßrigen Medium, das durch die Lipiddoppelschicht nach außen abgegrenzt ist. Je nach Lipophilie oder Hydrophilie können Wirkstoffe entweder in der Lipiddoppelschicht oder im wäßrigen Innenvolumen der Liposomen eingeschlossen und im Organismus über die Körperflüssigkeiten transportiert und verteilt werden.

Aufgrund ihrer Struktur dienen Liposomen in der Biochemie und Molekularbiologie als Modelle für Membranen. In den vergangenen Jahren sind außerdem zahlreiche Arbeiten über die Eigenschaften von Liposomen und ihre Verwendung als Arzneistoffträger veröffentlicht worden (vgl. z.B. H. Schreiner und M. Raeder-Schikorr, Pharmazie in unserer Zeit 11 (1982), 97-108). Bisher veröffentlichte Tierversuche zeigen generell, daß Leber und Milz bezüglich der Aufnahme von Liposomen über andere Organe dominieren. Etwa 8 % der Liposomen werden nach 1 Stunde und etwa 15 % nach 24 Stunden in der Leber vorgefunden.

Die mögliche Verwendung von Liposomen in der Medizin zielt im wesentlichen auf die selektive Behandlung von Krankheiten ab. Die gewünschten Wirkungen des in den Liposomen eingeschlossenen Wirkstoffes sollen gefördert werden, die unerwünschten Wirkungen dagegen vermindert werden. Auf diese Weise soll eine Verbesserung des therapeutischen Index erreicht werden.

Aus der DE-OS 40 13 632.9 sind Liposomen bekannt, die mindestens 1 Mol-% einer eine positive Überschußladung aufweisenden Verbindung enthalten.

Ein Nachteil der Verabreichung bekannter Liposomen besteht jedoch darin, daß die Aufnahme in der Leber relativ begrenzt ist und daß die Liposomen lange im Blut zirkulieren können. Dies gilt insbesondere für Liposomen, die aus Phospholipiden, wie etwa Lecithin, sowie Cholesterin zusammengesetzt sind. Auf diese Weise wird der in den Liposomen enthaltene Wirkstoff über den Körper verteilt, was wiederum zu einem vermehrten Auftreten von unerwünschten Nebenwirkungen des Wirkstoffes führen kann.

Eine Aufgabe der vorliegenden Erfindung bestand somit darin, neue Liposomen bereitzustellen, die eine erhöhte Leberaufnahme gegenüber Liposomen des Standes der Technik zeigen.

Die Lösung erfolgt durch Bereitstellen von einen pharmazeutischen Wirkstoff enthaltenden Liposomen, welche dadurch gekennzeichnet sind, daß sie 30 bis 50 Mol-% Cholesterin, 49 bis 56 Mol-% Phospholipide und 1 bis 14 Mol-% von einer oder mehreren Verbindungen der allgemeinen Formel I 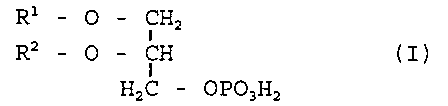
oder deren Salze auf Basis der gesamten Lipidbestandteile der Liposomen enthalten, wobei R¹ und R² gleich oder verschieden sein können und Wasserstoff, C₁-C₄ Alkylgruppen oder gesättigte oder ungesättigte C₈-C₂₄-Acylgruppen darstellen, die gegebenenfalls verzweigt oder/und substituiert sein können, vorausgesetzt, daß mindestens einer der Reste R¹ und R² eine Acylgruppe wie oben definiert ist, und erhältlich sind, indem man 1 bis 14 Mol-% Verbindungen der allgemeinen Formel I mit den weiteren Lipidbestandteilen der Liposomen in einer Menge, die zusammen mit den Verbindungen der allgemeinen Formel I 100 Mol-% ergibt, in einem geeigneten Lösungsmittel löst, das Lösungsmittel im Vakuum entfernt und den verbleibenden feinverteilten Lipidfilm mit einer wäßrigen Pufferlösung unter Bildung einer Lipidsuspension aufnimmt und vortempert, wobei man zum Einschluß wasserunlöslicher Wirkstoffe diese zusammen mit den Lipidbestandteilen löst und zum Einschluß wasserlöslicher Wirkstoffe diese als wäßrige Lösung dem Lipidfilm zusetzt, die Suspension durch Auspressen unter erhöhtem Druck aus einer Druckzelle in eine Liposomensuspension überführt und sterilisiert.

Die obigen Verbindungen (I) liegen bei physiologischen Bedingungen im allgemeinen als teilweise protonierte Anionen vor, so daß die erfindungsgemäßen Liposomen eine negative Überschußladung aufweisen. Der Anteil der negativ geladenen Trägersubstanzen gemäß Formel (I) beträgt vorzugsweise 3 bis 10 Mol-% auf Basis der gesamten Lipidbestandteile der Liposomen. Eine Erhöhung des Anteils von Verbindungen der Formel (I) über 15 % hinaus führt zu Löslichkeitsproblemen, die bis zur Ausflockung gehen können. Daher wird in der Regel der bevorzugte Bereich eingehalten.

Es sind solche Verbindungen der Formel (I) bevorzugt, bei denen entweder beide Reste C₈-C₂₄ Acylgruppen darstellen oder ein Rest Wasserstoff und der andere Rest eine C₈-C₂₄ Acylgruppe darstellt.

Besonders bevorzugt sind solche Verbindungen, bei denen R¹ eine C₁₄-C₂₄ Acylgruppe und R² Wasserstoff ist. Wiederum bevorzugte Beispiele aus dieser Gruppe sind 1-Stearoyl-sn-glycero-3-phosphorsäure, 1-Palmitoyl-sn-glycero-3-phosphorsäure, 1-Myristoyl-sn-glycero-3-phosphorsäure und 1-Erucoyl-sn-glycero-3-phosphorsäure sowie deren Salze. Diese Phosphatidsäuren werden vorzugsweise in Form von Salzen, z.B. den Monoalkalimetallsalzen, insbesondere den Mononatriumsalzen eingesetzt.

In einer weiteren besonders bevorzugten Verbindungsklasse sind R¹ und R² C₁₄-C₂₄ Acylgruppen. Wiederum bevorzugte Verbindungen aus dieser Klasse sind 1,2-Distearoyl-sn-glycero-3-phosphorsäure, 1,2-Dipalmitoyl-sn-glycero-3-phosphorsäure, 1,2-Dimyristoyl-sn-glycero-3-phosphorsäure und 1,2-Dierucoyl-sn-glycero-3-phosphorsäure sowie deren Salze. Auch diese Phosphatidsäuren werden vorzugsweise in Form ihrer Salze, z.B. in Form ihrer Monoalkalimetallsalze, insbesondere als Mononatriumsalze eingesetzt.

Überraschenderweise wurde festgestellt, daß ein Liposom, das eine negativ geladene Trägersubstanz gemäß Formel (I) enthält, erhebliche Vorteile gegenüber bekannten Liposomen aufweist, die ohne Verwendung von Verbindungen gemäß Formel (I) hergestellt worden sind.

Bei Verabreichung von Liposomen des Standes der Technik wurde nämlich festgestellt, daß deren Aufnahme in der Leber relativ begrenzt ist und daß sie daher ziemlich lange im Blut zirkulieren. Bei erfindungsgemäßen Liposomen wurde hingegen überraschend eine wesentlich bessere Leberaufnahme gefunden, was wiederum eine starke Verringerung von Nebenwirkungen der in den Liposomen eingeschlossenen pharmazeutischen Wirkstoffe in anderen Organen zur Folge hat.

Dies wurde insbesondere für das Anthracyclin-Antibiotikum Doxorubicin untersucht. Diese Substanz wird bereits seit etwa 25 Jahren klinisch verwendet. Seine Anwendung als Antitumormittel bei der Behandlung fester Tumoren, Leukämien und Lymphomen (Bonadonna et al., Cancer Res. 30 (1970), 2572-2582; Middleman et al., Cancer 28 (1971), 844-850; Tan et al., Cancer 32 (1973), 9-17) war jedoch aufgrund seiner hohen Herztoxizität (Lefrak et al., Cancer 32 (1973), 302-314; Rinehart et al., Ann.Internal.Med. 81 (1974), 475-478; von Hoff et al., Ann.Internal.Med. 91 (1979), 710-717) sehr begrenzt.

Bei den Arbeiten, die zur vorliegenden Erfindung führten, wurde nun durch Experimenten an Mäusen festgestellt, daß erfindungsgemäße Liposomen, die Doxorubicin als Wirkstoff enthalten, einerseits eine vergleichbare Plasmastabilität wie bekannte Liposomen zeigen, andererseits aber überraschenderweise sich von diesen in der Pharmakokinetik stark unterscheiden. Bei Verwendung der erfindungsgemäßen Liposomen wurden erheblich geringere Mengen an Doxorubicin im Plasma, in der Lunge, in der Niere und insbesondere auch im Herzen festgestellt. In der Leber, dem gewünschten Zielorgan der Liposomen wurde hingegen bei Verwendung der erfindungsgemäßen Liposomen eine erheblich höhere Konzentration an Doxorubicin als bei Verwendung von Liposomen des Standes der Technik oder bei Verwendung von freiem Doxorubicin festgestellt.

Ein weiterer überraschender Vorteil der erfindungsgemäßen Liposomen besteht darin, daß sich die Metabolisierung des darin enthaltenen Wirkstoffes von der Metabolisierung eines ohne Liposomen verabreichten Wirkstoffes unterscheidet. So führt eine Verabreichung von Doxorubicin in Form einer handelsüblichen Zubereitung (ohne Liposomen) dazu, daß bereits nach 1 Stunde in der Leber nur noch 20 % des Doxorubicins vorhanden sind, während der Rest bereits metabolisiert wurde. Die Verabreichung des gleichen Wirkstoffes in den erfindungsgemäßen Liposomen führt dagegen unter sonst gleichen Bedingungen nach 1 Stunde noch zu 97 % des Wirkstoffes in nichtmetabolisierter Form, so daß hier eine Metabolisierung von nur 3 % vorliegt. Im Gegensatz dazu sind bei handelsüblichen Zusammensetzungen (ohne Liposomen) in der Leber bereits nach 1 Stunde ca. 80 % des Wirkstoffes metabolisiert worden.

Die erfindungsgemäßen Liposomen enthalten neben Verbindungen der allgemeinen Formel (I) noch 30 bis 50 Mol-% Cholesterin und 49 bis 56 Mol-% Phospholipide als weitere Lipidbestandteile. Besonders bevorzugt sind ungeladene Phospholipide. Unter dem Begriff "ungeladene Phospholipide" sind solche Phospholipide zu verstehen, die nach außen ungeladen sind (d.h. der Begriff umfaßt solche Phospholipide, die intramolekulare Zwitterionen sind). Eine bevorzugte Klasse von Phospholipiden in den erfindungsgemäßen Liposomen sind Phosphoglyceride, d.h. Verbindungen, die eine Glyceringruppierung enthalten, wobei 2 Hydroxylgruppen des Glycerins durch Fettsäuregruppen (vorzugsweise C₈-C₂₄ Acylgruppen) und die dritte Hydroxylgruppe durch einen phosphorylierten Alkohol verestert ist. Beispiele für besonders geeignete Alkohole sind solche, die eine positive Ladung tragen, wie etwa Ethanolamin und Cholin. Besonders bevorzugt sind Phospholipide, bei denen es sich um Lecithine der allgemeinen Formel (II) 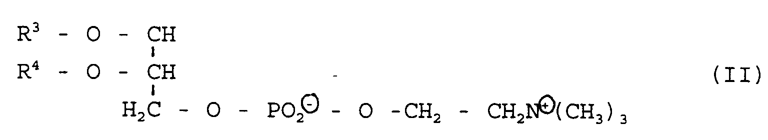
handelt, wobei R³ und R⁴ gleich oder verschieden sein können und gesättigte oder ungesättigte C₈-C₂₄ Acylgruppen darstellen, die gegebenenfalls verzweigt oder/und substituiert sein können. Vorzugsweise sind R³ und R⁴ C₁₄-C₂₄ Acylgruppen. Besonders bevorzugt sind R³ und R⁴ Stearoyl-, Palmitoyl-, Myristoyl- oder Erucoylreste.

Die Komponenten der erfindungsgemäßen Liposomen sind entweder kommerziell erhältlich oder nach bekannten Verfahren herstellbar. Lecithine der Formel (II) können beispielsweise nach Woolley und Eibl, Chem.Phys.Lipids 47 (1988), 55-62 und Eibl und Woolley, Chem.Phys.Lipids 41 (1986), 53-63 hergestellt werden. Weiterhin ist die Herstellung von Phosphoglyceriden in der DE-OS 32 25 213.7 und darin zitierten Literaturstellen beschrieben. Verbindungen der allgemeinen Formel (I) können beispielsweise aus den entsprechenden Lecithinen durch Abspaltung des Cholinrests mit Phospholipase D hergestellt werden (Eibl und Kovatchev, Methods Enzymol. 72 (1981), 632-639).

Zur Herstellung der erfindungsgemäßen Liposomen werden die einzelnen Lipidbestandteile der Liposomen, d.h. 1 bis 14 Mol-% Verbindungen der allgemeinen Formel (I) und die weiteren Lipidbestandteile in einer Menge, die zusammen mit den Verbindungen der Formel (I) 100 Mol-% ergibt, in einem geeigneten Lösungsmittel, vorzugsweise unter Erwärmen gelöst, um eine homogene Vermischung der Komponenten zu erreichen. Das Lösungsmittel wird im Vakuum entfernt und der feinverteilte Lipidfilm wird mit einer wäßrigen Pufferlösung (als wäßrige Pufferlösung können alle physiologisch verwendbaren Lösungen eingesetzt werden) versetzt. Anschließend wird das Gemisch unter leichtem Rühren ca. 1 Stunde lang auf einer Temperatur gehalten, die im allgemeinen etwa 5°C über der Hauptumwandlungstemperatur der Lipide liegt, z.B. bei 50°C.

Diese vorgetemperte Lipidsuspension wird dann durch geeignete Maßnahmen auf an sich bekannte Weise, z.B. in der Druckzelle einer French-Press (Firma Aminco, Silverspring, USA), in die Liposomen überführt. Die French-Press besteht aus einer hydraulischen Presse und einer Standarddruckzelle aus Stahl. Nach Verschließen der Druckzelle wird der Druck erhöht und die resultierende Liposomendispersion unter konstantem Druck durch einen schmalen Auslaß gepreßt. Der Vorgang wird mindestens dreimal wiederholt. Nach Zentrifugation der Liposomendispersion wird der Überstand vom Sediment entfernt. Er enthält die Liposomen, die nun für die verschiedenen Verwendungen und Untersuchungen, z.B. zur Herstellung von Liposomen, die einen oder mehrere pharmazeutische Wirkstoffe enthalten, zur Verfügung stehen. Die erfindungsgemäßen Liposomen können jedoch ebenso nach anderen Verfahren hergestellt werden.

Eine pharmazeutische Zubereitung, die erfindungsgemäße Liposomen und in den Liposomen eingeschlossen einen oder mehrere pharmazeutische Wirkstoffe enthält, läßt sich mit pharmazeutisch üblichen Verdünnungs-, Hilfs-, Träger- und Füllstoffen gewinnen.

Als Wirkstoffe können in der Regel alle Wirkstoffe verwendet werden, die sich mittels Liposomen überhaupt einschließen lassen. Bevorzugte Wirkstoffgruppen sind einerseits Cytostatika, insbesondere Anthracyclin-Antibiotika, wie etwa Doxorubicin, Epirubicin oder Daunomycin, wobei Doxorubicin besonders bevorzugt ist. Weitere bevorzugte Cytostatika sind Idarubicin, Hexadecylphosphocholin, 1-Octadecyl-2-methyl-rac-glycero-3-phosphocholin, 5-Fluoruracil, cis-Platinkomplexe wie Carboplatin und Novantron.

Weiterhin bevorzugte Wirkstoffgruppen sind immunmodulierende Substanzen wie etwa Cytokine, wobei unter diesen wiederum die Interferone und insbesondere das α-Interferon besonders bevorzugt sind, antimykotisch wirksame Substanzen (z.B. Amphotericin B) und Wirkstoffe gegen Protozoenerkrankungen (Malaria, Trypanosomen- und Leishmanien-Infektionen). Ebenfalls bevorzugt ist Taxol als Wirkstoff.

Bei Verwendung von erfindungsgemäßen Liposomen zur Herstellung eines Antitumormittels ist der Wirkstoff besonders bevorzugt Doxorubicin.

Bei Verwendung der erfindungsgemäßen Liposomen zur Herstellung eines Mittels zur Beeinflussung der Zellproliferation ist der Wirkstoff ein Cytokin, besonders bevorzugt α-Interferon.

Zur Herstellung einer pharmazeutischen Zusammensetzung, die einen oder mehrere Wirkstoffe, die in den erfindungsgemäßen Liposomen eingeschlossen sind, enthält, wird wie folgt verfahren:
Zum Einschluß wasserunlöslicher Substanzen wird der Wirkstoff zusammen mit den Lipiden in einem geeigneten Lösungsmittel, wie etwa Methylenchlorid oder Chloroform, gelöst, daran anschliessend wird nach dem oben beschriebenen Verfahren zur Herstellung der Liposomen gearbeitet.

Zum Einschluß wasserlöslicher Substanzen wird, wie zuvor beschrieben, der Lipidfilm mit einer Lösung versetzt, die jetzt aber den wasserlöslichen Wirkstoff enthält. Anschließend wird, wie zuvor beschrieben, weiterverfahren. Der Überstand nach der Zentrifugation enthält zusätzlich zu den gefüllten Liposomen auch den nicht eingeschlossenen wasserlöslichen Wirkstoff. Dieser freie Wirkstoffanteil kann von dem in Liposomen eingeschlossenen Anteil z.B. durch Diafiltration abgetrennt werden. Vor Verwendung der Liposomen wird vorzugsweise weiterhin eine Sterilfiltration mit Membranfiltern durchgeführt (Firma Sartorius, Porenweiten 0,2 µm).

Die Erfindung wird nachfolgend in den Beispielen weiter veranschaulicht.

### Beispiel 1

### Herstellung von Liposomen

1,2-Dipalmitoyl-sn-glycero-3-phosphocholin (DPPC) wurde von Sygena LTD (Liestal, CH) und Cholesterin (Ch) von Fluka (Buchs, CH) bezogen. 1,2-Distearoyl-sn-glycero-3-phosphocholin (DSPC) wurde über 1,2-Distearoyl-sn-glycerin hergestellt (Eibl und Kovatchev, Chem.Phys.Lipids 41 (1986), 53-63). 1,2-Distearoyl-sn-glycero-3-phosphat (DSPA) wurde durch Hydrolyse von 1,2-Distearoyl-sn-glycero-3-phosphocholin mit Phospholipase D erzeugt (Eibl und Woolley, Methods Enzymol. 72 (1981), 632-639). Hydriertes Soja-Phosphatidylinositol (HPI) wurde aus Sojalecithin (P5638), bezogen von Sigma Chemie GmbH (Steinheim, BRD), hergestellt. Sojalecithin enthält etwa 10 % Phosphatidylinositol, das durch Silicagelchromatographie isoliert wurde (Woolley und Eibl, Chem.Phys.Lipids 47 (1988), 55-62). Das resultierende Produkt wurde in 100 ml CHCl₃/CH₃OH (1:1, v/v) mit 10 % Pd/C-Katalysator hydriert.

Es wurden kleine unilamellare Liposomen mit den folgenden Zusammensetzungen (auf molarer Basis) hergestellt: DPPC/Ch (6:4); DSPC/DSPA/Ch (5:1:4); DPPC/HPI/Ch (5:1:4). 5 mmol der Lipidsuspensionen wurden hierzu in 50 ml Zitronensäurepuffer (300 mmol/l, pH 4) 3 Stunden lang in einem Rotationsverdampfer bei Temperaturen 5°C überhalb der Phasenübergangstemperatur (T_{M}) des Phospholipids mit der höchsten T_{M} mechanisch dispergiert. Aus dieser homogenen Lösung wurden Liposomen unter Verwendung einer French-Press bei 20.000 psi hergestellt. Die resultierenden Liposomen wurden 30 Minuten lang bei 30.000 g zentrifugiert. Darin wurden 100 mg pulverförmiges Doxorubicin zugegeben und bei pH 8 durch die Beladungsprozedur von Mayer et al. (Biochim.Biophys.Acta 857 (1986), 123-126) eingeschlossen. Nicht in die Liposomen eingeschlossenes freies Doxorubicin wurde durch Diafiltration (Polysulfon Ultrasart Modul, Ausschlußgrenze 20 kD, Sartocon Mini SM 17521, Sartorius, BRD) entfernt. Alle Liposomen wurden vor der Verwendung sterilfiltriert.

### Beispiel 2

### Behandlung von Mäusen mit Doxorubicin-haltigen Liposomen

Weibliche NMR-I Mäuse (28-32 g, 6-8 Wochen) wurden von der Tierzuchtanstalt Hannover, BRD bezogen. In die Schwanzvene der Mäuse wurden jeweils gleiche Dosen (5,7 mg/kg) injiziert. Jede Behandlungsgruppe bestand aus 3 Tieren. Nach 1, 24 und 72 Stunden wurden die Mäuse mit CO₂ anästhesiert. Blut wurde aus den Mäusen durch Herzpunktion gewonnen und in heparinisierten Eppendorf-Gefäßen aufbewahrt. Gewebeproben aus den Mäusen (Leber, Herz etc.) wurden in 0,9 %iger NaCl-Lösung gespült und bei -70°C aufbewahrt.

Die Plasmaproben wurden nach der Prozedur von Mross et al. (J.Chromatograph. 530 (1990), 192-199) analysiert. Hierzu wurden 100 µl Plasma auf aktivierte Bond Elut C18-Säulen (ICT, Frankfurt, BRD) geladen und mit 4 ml Puffer (0,02 mol/l Na-Phosphat, pH 3,9) gespült. Die Säulen wurden 10 Minuten lang bei 10 mbar getrocknet. Doxorubicin und seine Metaboliten wurden mit 4 ml Methanol/Chloroform (1:1, v/v) eluiert. Das Eluat wurde unter Stickstoff zur Trockene eingedampft. Der Rückstand wurde in 50 µl (0,02 mol/l Na-Phosphat/Acetonitril, pH 3,9; 7:3, v/v) aufgenommen und über HPLC analysiert, wobei eine 4,6 mm x 25 cm Merck Lichrocart C 18 RP 5 µm Säule verwendet wurde. Die Flußrate war 1,5 ml/min. Die Exzitationswellenlänge war 480 nm und die Emissionswellenlänge war 580 nm.

Zum Nachweis von Doxorubicin und seinen Metaboliten in den einzelnen Organen wurden die Gewebeproben (jeweils etwa 200 mg) mit einem Mikro-Dismembrator (Braun Melsungen AG, BRD) homogenisiert. Doxorubicin und seine Metaboliten wurden mit einer Extraktionslösung bei pH 3 (16,5 % w/v AgNO₃ in Acetonitril/Wasser; 6:4, v/v) behandelt, um die Anthracycline aus einem Komplex mit DNA freizusetzen und die Proteine zu fällen. Nach Zugabe eines inneren Standards (Epirubicin) und Zentrifugation wurde der Überstand direkt auf die HPLC-Säule gegeben.

Die DPPC/Ch-Liposomen hatten einen mittleren Durchmesser von 64 nm, die DPPC/HPI/Ch-Liposomen hatten einen mittleren Durchmesser von 91 nm und die erfindungsgemäßen DSPC/DSPA/Ch-Liposomen hatten einen mittleren Durchmesser von 88 nm.

Das mittlere Verhältnis von Doxorubicin zu Phospholipid war 170 µg/µmol für DPPC/Ch-Liposomen (95 % Einschluß),78 µg/µmol DSPC/DSPA/Ch-Liposomen (65 % Einschluß) und 65 µg/µmol für DPPC/HPI/Ch-Liposomen (71 % Einschluß). Die drei Liposomentypen waren im Plasma für mindestens 24 Stunden stabil. Auch nach 3 monatiger Lagerung bei 4°C konnte kein Austritt von Doxorubicin aus den Liposomen gefunden werde.

Die Pharmakokinetik von Doxorubicin in Abhängigkeit der Zubereitungsform ergab folgende Ergebnisse: Nach 1 Stunde war der Plasmawert bei Zugabe von freiem Doxorubicin (ohne Liposomen) 0,5 µg/ml und nur Spuren von Doxorubicin konnten nach 24 Stunden gefunden werden. Die DPPC/Ch- und CPPC/HPI/Ch-Liposomen zeigten sehr hohe Plasmaspiegel nach 1 Stunde (59,3 bzw. 42,9 µg/ml) und zeigten auch bis zu 24 Stunden relativ hohe Spiegel (5,5 bzw. 4,9 µg/ml). Hingegen konnten bei den erfindungsgemäßen DSPC/DSPA/Ch-Liposomen nur 7,7 µg/ml Doxorubicin im Plasma nach 1 Stunde nachgewiesen werden. Nach 24 Stunden konnten nur Spuren von Doxorubicin gefunden werden.

Im Herzen war der Spiegel an freiem Doxorubicin nach 1 Stunde 13 µg/g, wohingegen bei den erfindungsgemäßen DSPC/DSPA/Ch-Liposomen nur 1,0 µg/g gefunden wurden. Die entsprechenden Werte für DPPC/Ch- und CPPC/HPI/Ch-Liposomen waren etwa 3,5 µg/g Herzgewebe. In der Lunge und in der Niere wurden nach 1 Stunde bei Verabreichung von Doxorubicin in DPPC/DSPA/Ch-Liposomen ebenfalls deutlich geringere Wirkstoffkonzentrationen als bei den anderen Verabreichungsformen gefunden.

In der Leber wurden ebenfalls Unterschiede in der Doxorubicin-Konzentration in Abhängigkeit von der Verabreichungsform gefunden. Nach 1 Stunde waren die entsprechenden Doxorubicinwerte wie folgt: 11,0 µg/g für freies Doxorubicin, 42,8 µg/g für erfindungsgemäße DSPC/DSPA/Ch-Liposomen, 30 µg/g für DPPC/HPI/Ch-Liposomen und 12,2 µg/g für DPPC/Ch-Liposomen. Die erfindungsgemäßen Liposomen zeigten auch nach 24 Stunden einen hohen Wert von 25,1 µg/g Doxorubicin in der Leber. Selbst nach 72 Stunden war die Konzentration von Doxorubicin in diesem Gewebe noch bemerkenswert hoch.

Zur Metabolisierung von Doxorubicin in der Leber wurde folgendes festgestellt: Nach 1 Stunde wurden bei Verabreichung von freiem Doxorubicin 23 % aktives Mittel gegenüber 77 % inaktiven Metaboliten (Aglycone) gefunden. Hingegen wurden 97 % aktives Doxorubicin gegenüber 3 % inaktiven Aglyconen für die erfindungsgemäßen DSPC/DSPA/Ch-Liposomen beobachtet. Die entsprechenden Werte waren 96 %:4 % für DPPC/Ch-Liposomen und 90 %:10 % für DPPC/Ch-Liposomen.

In der Milz wurde nach 1 Stunde - wie bei der Leber - eine erhöhte Aufnahme von Doxorubicin bei Verwendung der DPPC/DSPA/Ch-Liposomen festgestellt.

Zusammenfassend wurde festgestellt, daß die Verabreichung von Doxorubicin in erfindungsgemäßen Liposomen der Verabreichung des Wirkstoffs in freier Form und auch der Verabreichung des Wirkstoffs in anderen Liposomen insbesondere aufgrund der höheren Gewebespezifität überlegen ist.

## Patentansprüche

1. Einen pharmazeutischen Wirkstoff enthaltende Liposomen,
**dadurch gekennzeichnet,**
daß sie 30 bis 50 Mol-% Cholesterin, 49 bis 56 Mol-% Phospholipide und 1 bis 14 Mol-% von einer oder mehreren Verbindungen der allgemeinen Formel I 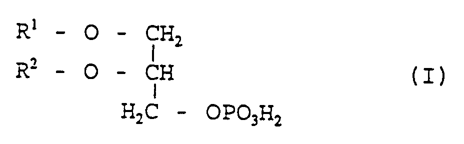 oder deren Salze auf Basis der gesamten Lipidbestandteilen der Liposomen enthalten,
wobei R¹ und R² gleich oder verschieden sein können und Wasserstoff, C₁-C₄ Alkylgruppen oder gesättigte oder ungesättigte C₈-C₂₄-Acylgruppen darstellen, die gegebenenfalls verzweigt oder/und substituiert sein können, vorausgesetzt, daß mindestens einer der Reste R¹ und R² eine Acylgruppe wie oben definiert ist, und erhältlich sind, indem man 1 bis 14 Mol-% Verbindungen der allgemeinen Formel I mit den weiteren Lipidbestandteilen der Liposomen in einer Menge, die zusammen mit den Verbindungen der allgemeinen Formel I 100 Mol-% ergibt in einem geeigneten Lösungsmittel löst, das Lösungsmittel im Vakuum entfernt und den verbleibenden feinverteilten Lipidfilm mit einer wäßrigen Pufferlösung unter Bildung einer Lipidsuspension aufnimmt und vortempert, wobei man zum Einschluß wasserunlöslicher Wirkstoffe diese zusammen mit den Lipidbestandteilen löst und zum Einschluß wasserlöslicher Wirkstoffe diese als wäßrige Lösung dem Lipidfilm zusetzt, die Suspension durch Auspressen unter erhöhtem Druck aus einer Druckzelle in eine Liposomensuspension überführt und diese sterilfiltriert.

2. Liposomen nach Anspruch 1,
**dadurch gekennzeichnet,**
daß sie 3 bis 10 Mol-% Verbindungen der Formel (I) oder deren Salze enthalten.

3. Liposomen nach Anspruch 1 oder 2,
**dadurch gekennzeichnet,**
daß R¹ eine C₁₄-C₂₄ Acylgruppe und R² Wasserstoff ist.

4. Liposomen nach Anspruch 3,
**dadurch gekennzeichnet,**
daß die Verbindungen der Formel (I) aus der Gruppe, bestehend aus 1-Stearoyl-sn-glycero-3-phosphorsäure, 1-Palmitoyl-sn-glycero-3-phosphorsäure, 1-Myristoyl-sn-glycero-3-phosphorsäure und 1-Erucoyl-sn-glycero-3-phosphorsäure sowie deren Salzen ausgewählt sind.

5. Liposomen nach Anspruch 4,
**dadurch gekennzeichnet,**
daß die Verbindungen die Mononatriumsalze der genannten Phosphatidsäuren sind.

6. Liposomen nach Anspruch 1 oder 2,
**dadurch gekennzeichnet,**
daß R¹ und R² C₁₄-C₂₄ Acylgruppen sind.

7. Liposomen nach Anspruch 6,
**dadurch gekennzeichnet,**
daß die Verbindungen der Formel (I) aus der Gruppe, bestehend aus 1,2-Distearoyl-sn-glycero-3-phosphorsäure, 1,2-Dipalmitoyl-sn-glycero-3-phosphorsäure, 1,2-Dimyristoyl-sn-glycero-3-phosphorsäure und 1,2-Dierucoyl-sn-glycero-3-phosphorsäure sowie deren Salzen ausgewählt sind.

8. Liposomen nach Anspruch 7,
**dadurch gekennzeichnet,**
daß die Verbindungen die Mononatriumsalze der genannten Phosphatidsäuren sind.

9. Liposomen nach einem der Ansprüche 1 bis 8,
**dadurch gekennzeichnet,**
daß die Phospholipide Phosphoglyceride sind.

10. Liposomen nach einem der Ansprüche 1 bis 9,
**dadurch gekennzeichnet,**
daß sie als Phospholipide Lecithine der allgemeinen Formel II 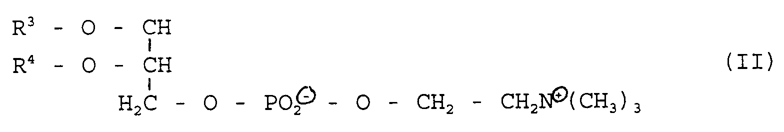 enthalten,
worin R³ und R⁴ gleich oder verschieden sein können und gesättigte oder ungesättigte C₈-C₂₄-Acylgruppen darstellen, die gegebenenfalls verzweigt oder/und substituiert sein können.

11. Liposomen nach Anspruch 10,
**dadurch gekennzeichnet,**
daß R³ und R⁴ C₁₄-C₂₄-Acylgruppen sind.

12. Liposomen nach Anspruch 11,
**dadurch gekennzeichnet,**
daß R³ und R⁴ Stearoyl-, Palmitoyl-, Myristoyl- oder Erucoylreste sind.

13. Liposomen nach einem der vorhergehenden Ansprüche,
**dadurch gekennzeichnet,**
daß die Verbindung der Formel (I) 1,2-Distearoyl-sn-glycero-3-phosphat und das Phospholipid 1,2-Distearoyl-sn-glycero-3-phosphocholin ist.

14. Liposomen nach einem der vorhergehenden Ansprüche,
**dadurch gekennzeichnet,**
daß der Wirkstoff ausgewählt aus der Gruppe Doxorubicin, Anthracyclinantibiotikum, Idarubicin, Carboplatin, Taxol und Cytokin.

## Claims

1. Liposomes containing a pharmaceutical active substance,
**wherein**
they contain 30 to 50 mol % cholesterol, 49 to 56 mol % phospholipids and 1 to 14 mol % of one or several compounds of the general formula I 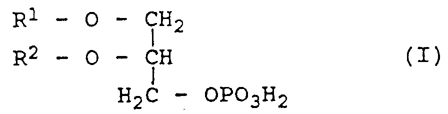 or salts thereof in relation to the total lipid components of the liposomes,
in which R¹ and R² can be the same or different and represent hydrogen, C₁-C₄ alkyl groups or saturated or unsaturated C₈-C₂₄ acyl groups which can, if desired, be branched or/and substituted provided that at least one of the residues R¹ and R² is an acyl group as defined above, and which can be obtained by dissolving 1 to 14 mol % of compounds of the general formula I together with the other lipid components of the liposomes in an amount that together with the compounds of the general formula I amounts to 100 mol % in a suitable solvent, removing the solvent in a vacuum and taking up the remaining finely dispersed lipid film with an aqueous buffer solution with formation of a lipid suspension and pre-heating, during which in order to entrap water-insoluble active substances, these are dissolved together with the lipid components and in order to entrap water-soluble active substances, these are admixed as an aqueous solution with the lipid film the suspension is converted into a liposome suspension by being pressed from a pressure cell under increased pressure and this is sterilized by filtration.

2. Liposomes as claimed in claim 1,
**wherein**
they contain 3 to 10 mol % of compounds of formula (I) or salts thereof.

3. Liposomes as claimed in claim 1 or 2,
**wherein**
R¹ is a C₁₄-C₂₄ acyl group and R² is hydrogen.

4. Liposomes as claimed in claim 3,
**wherein**
the compounds of formula (I) are selected from the group comprising 1-stearoyl-sn-glycero-3-phosphoric acid, 1-palmitoyl-sn-glycero-3-phosphoric acid, 1-myristoyl-sn-glycero-3-phosphoric acid and 1-erucoyl-sn-glycero-3-phosphoric acid as well as salts thereof.

5. Liposomes as claimed in claim 4,
**wherein**
the compounds are the monosodium salts of the said phosphatidic acids.

6. Liposomes as claimed in claim 1 or 2,
**wherein**
R¹ and R² are C₁₄-C₂₄ acyl groups.

7. Liposomes as claimed in claim 6,
**wherein**
the compounds of formula (I) are selected from the group comprising 1,2-distearoyl-sn-glycero-3-phosphoric acid, 1,2-dipalmitoyl-sn-glycero-3-phosphoric acid, 1,2-dimyristoyl-sn-glycero-3-phosphoric acid and 1,2-dierucoyl-sn-glycero-3-phosphoric acid as well as the salts thereof.

8. Liposomes as claimed in claim 7,
**wherein**
the compounds are the monosodium salts of the said phosphatidic acids.

9. Liposomes as claimed in one of the claims 1 to 8,
**wherein**
the phospholipids are phosphoglycerides.

10. Liposomes as claimed in one of the claims 1 to 9,
**wherein**
they contain lecithins of the general formula II as phospholipids 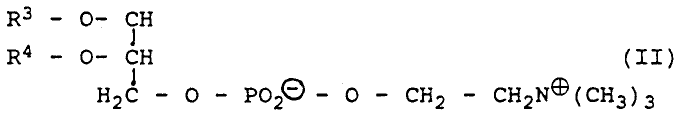 in which R³ and R⁴ can be the same or different and represent saturated or unsaturated C₈-C₂₄ acyl groups which if desired, can be branched or/and substituted.

11. Liposomes as claimed in claim 10,
**wherein**
R³ and R⁴ are C₁₄-C₂₄ acyl groups.

12. Liposomes as claimed in claim 11,
**wherein**
R³ and R⁴ are stearoyl, palmitoyl, myristoyl or erucoyl residues.

13. Liposomes as claimed in one of the previous claims,
**wherein**
the compound of formula (I) is 1,2-distearoyl-sn-glycero-3-phosphate and the phospholipid is 1,2-disteraroyl-sn-glycerol-3-phosphocholine.

14. Liposomes as claimed in one of the previous claims,
**wherein**
the active substance is selected from the group doxorubicin, anthracyclin antibiotic, idarubicin, carboplatin, taxol and cytokine.

## Revendications

1. Liposomes contenant une substance active pharmaceutique, caractérisés en ce qu'ils contiennent 30 à 50 % en moles de cholestérol, 49 à 56 % en moles de phospholipides et 1 à 14 % en moles d'un ou plusieurs composés de formule générale I 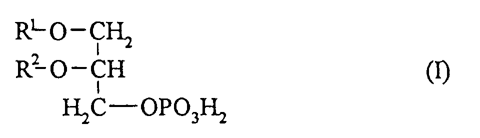 ou de leurs sels, par rapport à l'ensemble des constituants lipidiques des liposomes, R¹ et R² pouvant être identiques ou différents et représentant l'hydrogène, des groupes alkyle en C₁-C₄ ou des groupes acyle en C₈-C₂₄ saturés ou insaturés qui peuvent éventuellement être ramifiés et/ou substitués, étant entendu qu'au moins l'un des restes R¹ et R² est un reste acyle tel que défini ci-dessus, et en ce qu'ils peuvent être obtenus par un procédé selon lequel on dissout dans un solvant convenable 1 à 14 % en moles de composés de formule générale I avec les autres constituants lipidiques des liposomes en une quantité formant 100 % en moles avec les composés de formule générale I, on élimine le solvant sous vide, on reprend le film lipidique finement divisé restant dans une solution aqueuse de tampon pour former une suspension de lipides et on effectue une cuisson préalable, l'inclusion des substances actives insolubles dans l'eau se faisant par dissolution de ces substances avec les constituants lipidiques, et l'inclusion des substances actives solubles dans l'eau s'effectuant par l'addition de ces substances, sous forme d'une solution aqueuse, au film lipidique, on transforme la suspension en une suspension de liposomes en la pressant sous haute pression dans une cellule de pression, et on effectue une filtration stérilisante de cette suspension.

2. Liposomes selon la revendication 1, caractérisés en ce qu'ils contiennent 3 à 10 % en moles de composés de formule (I) ou de leurs sels.

3. Liposomes selon la revendication 1 ou 2, caractérisés en ce que R¹ est un groupe acyle en C₁₄-C₂₄ et R² est un atome d'hydrogène.

4. Liposomes selon la revendication 3, caractérisés en ce que les composés de formule (I) sont choisis dans le groupe constitué par l'acide 1-stéaroyl-sn-glycéro-3-phosphorique, l'acide 1-palmitoyl-sn-glycéro-3-phosphorique, l'acide 1-myristoyl-sn-glycéro-3-phosphorique et l'acide 1-érucoyl-sn-glycéro-3-phosphorique et leurs sels.

5. Liposomes selon la revendication 4, caractérisés en ce que les composés sont les sels monosodiques des acides phosphatidiques cités.

6. Liposomes selon la revendication 1 ou 2, caractérisés en ce que R¹ et R² sont des groupes acyle en C₁₄-C₂₄.

7. Liposomes selon la revendication 6, caractérisés en ce que les composés sont choisis dans le groupe constitué par l'acide 1,2-distéaroyl-sn-glycéro-3-phosphorique, l'acide 1,2-dipalmitoyl-sn-glycéro-3-phosphorique, l'acide 1,2-dimyristoyl-sn-glycéro-3-phosphorique et l'acide 1,2-diérucoyl-sn-glycéro-3-phosphorique et leurs sels.

8. Liposomes selon la revendication 7, caractérisés en ce que les composés sont les sels monosodiques des acides phosphatidiques cités.

9. Liposomes selon l'une des revendications 1 à 8, caractérisés en ce que les phospholipides sont des phosphoglycérides.

10. Liposomes selon l'une des revendications 1 à 9, caractérisés en ce qu'ils contiennent en tant que phospholipides des lécithines de formule générale II 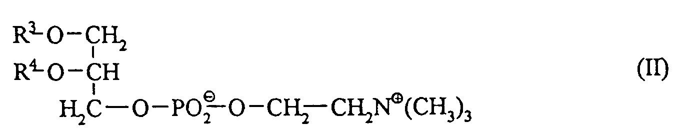 dans laquelle R³ et R⁴ peuvent être identiques ou différents et représentent des groupes acyle saturés ou insaturés en C₈-C₂₄, qui peuvent éventuellement être ramifiés et/ou substitués.

11. Liposomes selon la revendication 10, caractérisés en ce que R³ et R⁴ sont des groupes acyle en C₁₄-C₂₄.

12. Liposomes selon la revendication 11, caractérisés en ce que R³ et R⁴ sont des restes stéaroyle, palmitoyle, myristoyle ou érucoyle.

13. Liposomes selon l'une des revendications précédentes, caractérisés en ce que le composé de formule (I) est un 1,2-distéaroyl-sn-glycéro-3-phosphate et le phospholipide est la 1,2-distéaroyl-sn-glycéro-3-phosphocholine.

14. Liposomes selon l'une des revendications précédentes, caracterisés en ce que le principe actif est choisi dans le groupe doxorubicine, l'antibiotique anthracycline, idarubicine, carboplatine, taxol et cytokine.
